# EUROPÄISCHE PATENTSCHRIFT

(11) **EP 1 261 580 B1**
(45) Veröffentlichungstag und Bekanntmachung des Hinweises auf die Patenterteilung: **19.11.2014**
(21) Anmeldenummer: 01929411.5
(22) Anmeldetag: 06.03.2001
(51) Int. Cl.: C07C 253/34, C07C 209/86

(54) **VERFAHREN ZUR ABTRENNUNG EINES AZEPIN-DERIVATS AUS EINER MISCHUNG ENTHALTEND EIN AMIN UND EIN AZEPIN-DERIVAT**
METHOD FOR SEPARATING AN AZEPINE DERIVATIVE OUT OF A MIXTURE CONTAINING AN AMINE AND AN AZEPINE DERIVATIVE
PROCEDE POUR SEPARER UN DERIVE D'AZEPINE D'UN MELANGE CONTENANT UNE AMINE ET UN DERIVE D'AZEPINE

(30) Priorität: 08.03.2000 DE 10010825
(43) Veröffentlichungstag der Anmeldung: 04.12.2002
(73) Patentinhaber: BASF SE, 67056 Ludwigshafen (DE)
(72) Erfinder: LUYKEN, Hermann, 67069 Ludwigshafen (DE); OHLBACH, Frank, 40219 Düsseldorf (DE)
(86) Internationale Anmeldenummer: PCT/EP2001/002494
(87) Internationale Veröffentlichungsnummer: WO 2001/066514

(56) Entgegenhaltungen:
- EP-A- 0 497 333
- EP-A- 0 502 439
- WO-A-96/20931
- WO-A-98/11060
- WO-A-98/34900
- WO-A-98/34911
- DE-A- 19 839 338
- US-A- 5 961 788

## Beschreibung

Verfahren zur destillativen Abtrennung eines Teiles oder der Gesamtheit eines Azepin-Derivats (III) ausgewählt aus der Gruppe bestehend aus, Tetrahydroazepin, 2-Aminoazepan, N-(2-Azepano)-1,6-Diaminohexan und N-(2-Azepano)-6-Aminocapronitril aus einer Mischung (II) enthaltend ein Azepin-Derivat (III) und ein Hexamethylendiamin (I) aus der partiellen oder vollständigen Hydrierung von Adipodinitril und Hydrierung des abgetrennten Azepin-Derivates (III) zur Herstellung von Hexamethylendiamin (I), dadurch gekennzeichnet, daß man die Destillation bei einer Sumpftemperatur von höchstens 120°C durchführt, wobei man das Azepin-Derivat (III) bei der Destillation überwiegend als Sumpfprodukt (VI) erhält und dieses Sumpfprodukt (VI) einer Hydrierung unterwirft zur Herstellung von Hexamethylendiamin (I), 6-Aminocapronitril oder eines Gemisches aus Hexamethylendiamin (I) und 6-Aminocapronitril einsetzt.

Mischungen enthaltend ein Amin und ein Azepin-Derivat fallen üblicherweise bei der Hydrierung von Nitrilen zu Aminen an.

Die vollständige Hydrierung von Adipodinitril (ADN) zu Hexamethylendiamin (HMD), sowie die partielle Hydrierung unter gleichzeitiger Herstellung von HMD und 6-Aminocapronitril (ACN), in Gegenwart eines Katalysators auf der Basis eines Metalls wie Nickel, Cobalt, Eisen, Rhodium oder Ruthenium ist allgemein bekannt, beispielsweise aus: K. Weissermel, H.-J. Arpe, Industrielle Organische Chemie, 3. Auflage, VCH Verlagsgesellschaft mbH, Weinheim, 1988, Seite 266, US-A 4 601 859, US-A 2 762 835, US-A 2 208 598, DE-A 848 654, DE-A 954 416, DE-A 42 35 466, US-A 3 696 153, DE-A 19500222, WO-A-92/21650 und DE-A-19548289.

Als Nebenprodukte entstehen unter anderem Azepin-Derivate wie N-(2-Azepano)-1,6-Diaminohexan und N-(2-Azepano)-6-Aminocapronitril, insbesondere 2-Aminoazepan und Tetrahydroazepin.

Diese Azepin-Derivate, die wegen ihrer Farbgebung und Verschlechterung der Produkteigenschaften unerwünschte Verunreinigungen der üblicherweise für die Herstellung von Kunstfasern verwendeten Amine darstellen, lassen sich nur mit erheblichem Aufwand von den Aminen abtrennen.

So ist aus GB-A-893 709 bekannt, einen Verweilzeitbehälter in die Rückflußleitung einer für die Reinigung von HMD verwendeten Destillationskolonne einzubauen.

GB-A-1 238 351 beschreibt die Abtrennung von HMD aus Mischungen, die HMD und Azepin-Derivate enthalten, durch Zugabe von Akalihydroxid-Gemischen.

Aus WO-A-99/48872 ist bekannt, Azepin-Derivate von Aminen bei Kopftemperaturen von 160 bis 250°C abzudestillieren. Nachteilig bei diesem Verfahren ist eine unbefriedigende Trennung.

Nachteilig bei den genannten Verfahren sind die Anwendung großer Behälter mit einer verschlechterten Regelbarkeit der Destillationskolonnen, die Bildung von Feststoffen, die zu Verstopfungen führen können und eine unbefriedigende Abtrennung der Azepin-Derivate.

Aufgabe der vorliegenden Erfindung war es daher, ein Verfahren bereitzustellen, das die Abtrennung eines Azepin-Derivats aus Mischungen enthaltend ein Amin und ein Azepin-Derivat, auf technisch einfache und wirtschaftliche Weise ermöglicht.

Demgemäß wurde das eingangs definierte Verfahren gefunden.

Als Amine I kommen aromatische Amine wie Benzylamin, aliphatische Amine wie cyclische Amine, beispielsweise Isophorondiamin oder vorzugsweise acyclische Amine, beispielsweise 1,4-Diaminobutan, insbesondere HMD oder ACN, sowie deren Gemische in Betracht.

Die Herstellung solcher Amine kann in an sich bekannter Weise erfolgen.

So kann HMD durch partielle oder vollständige katalytische Hydrierung mit einem molekularen Wasserstoff enthaltenden Gas von ADN zu HMD oder Mischungen, die HMD und ACN enthalten, erhalten werden.

Als Katalysatoren können bei dieser Hydrierung vorteilhaft solche auf der Basis eines Metalles ausgewählt aus der Gruppe bestehend aus Ruthenium, Rhodium, Nickel, Cobalt, vorzugsweise Eisen eingesetzt werden, wobei die Katalysatoren weitere Elemente als Promotoren enthalten können. Im Falle von Katalysatoren auf der Basis von Eisen kommen als Promotoren insbesondere ein oder mehrere, wie zwei, drei, vier oder fünf Elemente ausgewählt aus der Gruppe bestehend aus Aluminium, Silizium, Zirkonium, Titan und Vanadium in Betracht.

Solche Katalysatoren sowie die Verfahrensbedingungen für die genannte Umsetzung sind beispielsweise in WO-A-96/20166, DE-A-19636768 und DE-A-19646436 beschrieben.

Als Azepin-Derivate III kommen insbesondere 2-Aminoazepan der Formel

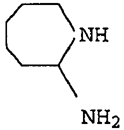

N-(2-Azepano)-1,6-Diaminohexan der Formel

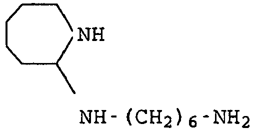

N-(2-Azepano)-6-Aminocapronitril 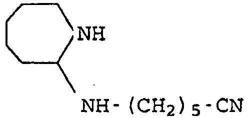 und THA der Formel 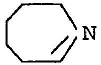 sowie deren Gemische in Betracht.

Die Azepin-Derivate (III) können in der Mischung (II) als einzelne Verbindungen oder als Addukte, beispielsweise an ein Amin (I), vorliegen, wobei diese Addukte im Sinne der vorliegenden Erfindung ebenfalls als Azepin-Derivate (III) bezeichnet werden.

Solche Azepin-Derivate sowie Verfahren zu deren Herstellung sind allgemein bekannt.

So können 2-Aminoazepan, N-(2-Azepano)-1,6-Diaminohexan und N-(2-Azepano)-6-Aminocapronitril und Tetrahydroazepin bei der partiellen katalytischen Hydrierung mit einem molekularen Wasserstoff enthaltenden Gas von ADN zu HMD oder Mischungen, die HMD und ACN enthalten, in der Regel in Mengen von 1 bis 10000 ppm bezogen auf das Gemisch nach den für die Herstellung der Amine (I) beschriebenen Verfahren in Mischungen (II) erhalten werden.
Ebenso können die genannten Azepin-Derivate durch Oxidation von Aminen, wie HMD und ACN, beispielsweise mit molekularen Sauerstoff enthaltenden Gasen, gebildet werden.

Erfindungsgemäß destilliert man bei Sumpftemperaturen von höchstens 120°C. Vorteilhaft destilliert man bei Sumpftemperaturen von mindestens 50°C, vorzugsweise mindestens 80°C.

Die Destillation kann man kontinuierlich durchführen.
Die Destillation kann man diskontinuierlich durchführen.

Setzt man als Amin (I) HMD und als Azepin-Derivat (III) eine oder mehrere Verbindungen ausgewählt aus der Gruppe bestehend aus AHI, HHA, AHHA und THA ein, so sollte der Destillationsdruck, gemessen am Sumpf der Destillations-Apparatur, 1 bis 300 mbar, vorzugsweise 5 bis 100 mbar, insbesondere 10 bis 60 mbar betragen.

Setzt man als Amin (I) ACN und als Azepin-Derivat (III) eine oder mehrere Verbindungen ausgewählt aus der Gruppe bestehend aus 2-Aminoazepan, N-(2-Azepano)-1,6-Diaminohexan und N-(2-Azepano)-6-Aminocapronitril und Tetrahydroazepin ein, so sollte der Destillationsdruck, gemessen am Sumpf der Destillations-Apparatur, 1 bis 200 mbar, vorzugsweise 5 bis 100 mbar, insbesondere 10 bis 40 mbar betragen.

Vorteilhaft erhält man Amin (I) bei der Destillation oberhalb des Zulaufs der Mischung (II) in die Destlillationsvorrichtung, insbesondere am Kopf der Destlillationsvorrichtung.

Vorteilhaft erhält man bei der Destillation ein Sumpfprodukt (VI) mit einem höheren Gewichtsanteil an Azepin-Derivat (III) als Mischung (II).

Für die Destillation kommen hierfür übliche Apparaturen in Betracht, wie sie beispielsweise in: Kirk-Othmer, Encyclopedia of Chemical Technology, 3.Ed., Vol. 7, John Wiley & Sons, New York, 1979, Seite 870-881 beschrieben sind, wie Siebbodenkolonnen, Glockenbodenkolonnen, Packungskolonnen oder Füllkörperkolonnen.

Die Destillation kann man in mehreren, wie 2 oder 3 Kolonnen, vorteilhaft einer einzigen Kolonne durchführen.

In einer bevorzugten Ausführungsform kann man die Destillation in zwei Stufen durchführen.

Dabei kann die erste Stufe aus mehreren, wie 2 oder 3 Kolonnen, vorteilhaft einer einzigen Kolonne bestehen. Dabei kann die zweite Stufe aus mehreren, wie 2 oder 3 Kolonnen, vorteilhaft einer einzigen Kolonne bestehen.

Vorteilhafterweise sollte der Druck in der ersten Stufe, gemessen im Sumpf, mindestens das 1,5 fache, insbesondere mindestens das doppelte, des Drucks in der zweiten Stufe, gemessen im Sumpf, betragen.

Vorteilhafterweise sollte dem Sumpf der ersten Stufe mindestens 20 Gew.-% der pro Zeiteinheit der ersten Stufe zugeführten Menge entnommen und der zweiten Stufe zugeführt werden.

Vorteilhafterweise kann das Kopfprodukt der zweiten Stufe in die erste Stufe zurückgeführt werden.

Vorteilhaft kann man dem Destillationsgemisch eine Verbindung (IV), deren Siedepunkt unter den Destillationsbedingungen über dem Siedepunkt des Amins (I) liegt, zugeben. Hierzu kommen insbesondere solche Verbindungen (V) in Betracht, die gegenüber dem Amin (I) unter den Destillationsbedingungen inert sind.

Als Verbindungen (IV) kommen Verbindungen aus der Gruppe der Aromaten, der Aliphaten, wie acyclische und cyclische Aliphaten, und aliphatisch-aromatische Verbindungen in Betracht. Diese Verbindungen können Substituenten tragen, wie eine Hydroxyl-, Keto-, Ester-, Alkyl-, Aryl, Cycloalkyl-, Arylalkyl-Gruppe, vorzugsweise eine Nitril- oder Amino-Gruppe, oder mehrere gleiche oder unterschiedliche solcher Gruppen.

Die Verbindung (IV) kann aus einer Verbindung oder Gemischen solcher Verbindungen bestehen.

Vorteilhaft sind solche Verbindungen (IV), die sich auf einfache Weise, wie durch Hydrierung beispielsweise mit einem molekularen Wasserstoff enthaltenden Gas in Gegenwart eines Katalysators, zu einer Mischung (V) enthaltend ein Amin (I) und ein Imin (III) oder insbesondere einer Mischung (II) umsetzen lassen.

Die bei dieser Umsetzung erhaltenen Produkte können in das erfindungsgemäße Verfahren vorteilhaft erneut eingesetzt werden.

Die Differenz der Siedepunkte zwischen dem Amin (I) und der Verbindung (IV) sollte unter den Destillationsbedingungen 1 bis 200°C, vorzugsweise 5 bis 100°C betragen.

Die Verbindung (IV) kann zu der Mischung (II) vor oder während der Destillation zugegeben werden.

Die Zugabe der Verbindung (IV) zu der Mischung (II) vor der Destillation kann in an sich bekannter Weise in üblichen Mischapparaturen erfolgen. Bei dieser Verfahrensführung kommt die Zugabe einer Mischung aus Mischung (II) und Verbindung (IV) in die Destillationsvorrichtung in Betracht.

Die Zugabe der Verbindung (IV) zu der Mischung (II) während der Destillation kann durch Einspeisen der Verbindung (IV) in die Destillationsapparatur vorzugsweise im Sumpfbereich erfolgen.

Die Destillation kann man vorteilhaft in Gegenwart von Hilfsstoffen, die die erfindungsgemäße destillative Trennung unterstützen, insbesondere in Gegenwart von Kohlendioxid, durchführen.

Während der Destillation sollte die Konzentration an Azepin-Derivat (III) im Sumpf, bezogen auf die im Sumpf vorhandene Mischung, höchstens 0,5 Gew.-%, vorzugsweise höchstens 0,2 Gew.-%, insbesondere höchstens 0,15 Gew.-% betragen.

Bei dem erfindungsgemäßen Verfahren erhält man üblicherweise den überwiegenden Teil an Azepin-Derivat (III) als Sumpfprodukt (VI). Dieses Sumpfprodukt (VI) enthält üblicherweise Azepin (III) in einer höheren Gewichts-konzentration als die zur Destillation nach dem erfindungsgemäßen Verfahren eingesetzte Mischung (II).

Sumpfprodukt (VI) kann man vorteilhaft in an sich bekannter Weise, beispielsweise nach den bereits für die Herstellung von HMD oder Mischungen enthaltend HMD und ACN genannten Verfahren, einer katalytischen Hydrierung unterwerfen, beispielsweise unter Erhalt eines Amins (I), wie HMD oder Mischungen, die HMD und ACN enthalten. Bei der Hydrierung kann Azepin-Derivat (III) zu organischen Verbindungen, wie Hexamethylenimin, umgesetzt werden, die im Gemisch mit Amin (I) eine Abtrennung von Amin (I) auf technisch einfache und wirtschaftliche Weise ermöglichen.

HMD und ACN stellen Vorstufen zur Herstellung von technisch wichtigen Polyamiden, wie Nylon 6 oder Nylon 6.6 dar.

### Beispiele

In den Beispielen sind Prozentangaben Gewichtsprozente, soweit nichts anderes angegeben.

### THA bedeutet Tetrahydroazepin.

Die Produktgemische wurden gaschromatographisch analysiert. Die Menge an THA wurde bei Konzentrationen unter 20 ppm polarographisch bestimmt.

### Beispiel

50 kg/h HMD mit einem THA-Gehalt von 71 ppm wurden einer Destillationskolonne mit 50 theoretischen Trennstufen gleichmäßig zugeführt und bei einem Rücklaufverhältnis von 1, einem Druck im Sumpf der Destillationskolonne von 73 mbar und einer Sumpftemperatur von 119.9°C 41 kg/h Kopfprodukt und 9 kg/h Sumpfprodukt der Destillationsvorrichtung entnommen.

Das Kopfprodukt enthielt neben HMD 12 ppm THA, das Sumpfprodukt 334 ppm THA.

### Vergleichsbeispiel

Es wurde verfahren wir im Beispiel mit dem Unterschied, daß der Druck im Sumpf 255 mbar und die Sumpftemperatur 153.5°C betrug. Das Kopfprodukt enthielt neben HMD 44 ppm THA, das Sumpfprodukt 172 ppm THA.

## Patentansprüche

1. Verfahren zur destillativen Abtrennung eines Teiles oder der Gesamtheit eines Azepin-Derivats (III) ausgewählt aus der Gruppe bestehend aus, Tetrahydroazepin, 2-Aminoazepan, N-(2-Azepano)-1,6-Diaminohexan und N-(2-Azepano)-6-Aminocapronitril aus einer Mischung (II) enthaltend ein Azepin-Derivat (III) und ein Hexamethylendiamin (I) aus der partiellen oder vollständigen Hydrierung von Adipodinitril und Hydrierung des abgetrennten Azepin-Derivates (III) zur Herstellung von Hexamethylendiamin (I), **dadurch gekennzeichnet, daß** man die Destillation bei einer Sumpftemperatur von höchstens 120°C durchführt, wobei man das Azepin-Derivat (III) bei der Destillation überwiegend als Sumpfprodukt (VI) erhält und dieses Sumpfprodukt (VI) einer Hydrierung unterwirft zur Herstellung von Hexamethylendiamin (I), 6-Aminocapronitril oder eines Gemisches aus Hexamethylendiamin (I) und 6-Aminocapronitril einsetzt.

2. Verfahren nach Anspruch 1, wobei man die Destillation bei einer Sumpftemperatur von höchstens 110°C durchführt.

3. Verfahren nach den Ansprüchen 1 oder 2, wobei während der Destillation die Konzentration an Azepin-Derivat (III) im Sumpf, bezogen auf die im Sumpf vorhandene Mischung, höchstens 0,5 Gew.-% beträgt.

4. Verfahren nach den Ansprüchen 1 bis 3, wobei man Hexamethylendiamin (I) oberhalb der Zugabestelle der Mischung (II) zu der Destillationsvorrichtung erhält.

5. Verfahren nach den Ansprüchen 1 bis 4, wobei man die Destillation in zwei Stufen durchführt, der Druck in der ersten Stufe, gemessen im Sumpf, mindestens das 1,5 fache des Drucks in der zweiten Stufe, gemessen im Sumpf, beträgt, dem Sumpf der ersten Stufe mindestens 20 Gew.-% der pro Zeiteinheit der ersten Stufe zugeführten Menge entnommen und der zweiten Stufe zugeführt wird und das Kopfprodukt der zweiten Stufe in die erste Stufe zurückgeführt wird.

6. Verfahren nach den Ansprüchen 1 bis 5, wobei die Destillation in Gegenwart von Kohlendioxid durchgeführt wird.

## Claims

1. A process for distillative removal of part or all of an azepine derivative (III) selected from the group consisting of tetrahydroazepine, 2-aminoazepan, N-(2-azepano)-1,6-diaminohexane and N-(2-azepano)-6-aminocapronitrile from a mixture (II) comprising an azepine derivative (III) and a hexamethylenediamine (I) from the partial or complete hydrogenation of adiponitrile and hydrogenation of the azepine derivative (III) removed to prepare hexamethylenediamine (I), which comprises conducting the distillation at a pot temperature of not more than 120°C, wherein the distillation provides said azepine derivative (III) predominantly as bottom product (VI) and said bottom product (VI) is subjected to a hydrogenation to prepare hexamethylenediamine (I), 6-aminocapronitrile or a mixture of hexamethylenediamine (I) and 6-aminocapronitrile.

2. The process according to claim 1, wherein the distillation is carried out at a pot temperature of not more than 110°C.

3. The process according to claim 1 or 2, wherein the concentration of azepine derivative (III) in the pot, based on the mixture present in the pot, is not more than 0.5% by weight during the distillation.

4. The process according to any of claims 1 to 3, wherein hexamethylenediamine (I) is obtained above the feed of mixture (II) to the distillation apparatus.

5. The process according to any of claims 1 to 4, wherein the distillation is carried out in two stages, the pressure in the first stage, measured in the pot, is at least 1.5 times the pressure in the second stage, measured in the pot, not less than 20% by weight of the amount fed into the first stage per unit time is removed from the pot of the first stage and fed to the second stage, and the overhead product of the second stage is recycled into the first stage.

6. The process according to any of claims 1 to 5, wherein the distillation is carried out in the presence of carbon dioxide.

## Revendications

1. Procédé de séparation par distillation d'une partie ou de la totalité d'un dérivé d'azépine (III) choisi dans le groupe constitué par la tétrahydroazépine, le 2-aminoazépane, le N-(2-azépano)-1,6-diaminohexane et le N-(2-azépano)-6-aminocapronitrile d'un mélange (II) contenant un dérivé d'azépine (III) et une hexaméthylènediamine (I) issu de l'hydrogénation partielle ou totale d'adipodinitrile, et d'hydrogénation du dérivé d'azépine (III) séparé pour la fabrication d'hexaméthylènediamine (I), **caractérisé en ce que** la distillation est réalisée à une température de fond d'au plus 120 °C, le dérivé d'azépine (III) étant principalement obtenu en tant que produit de fond (VI) lors de la distillation et ce produit de fond (VI) étant soumis à une hydrogénation pour la fabrication d'hexaméthylènediamine (I), de 6-aminocapronitrile ou d'un mélange d'hexaméthylènediamine (I) et de 6-aminocapronitrile.

2. Procédé selon la revendication 1, dans lequel la distillation est réalisée à une température de fond d'au plus 110 °C.

3. Procédé selon les revendications 1 ou 2, dans lequel la concentration en dérivé d'azépine (III) dans le fond pendant la distillation, par rapport au mélange présent dans le fond, est d'au plus 0,5 % en poids.

4. Procédé selon les revendications 1 à 3, dans lequel l'hexaméthylènediamine (I) est obtenue au-dessus de l'emplacement d'ajout du mélange (II) dans le dispositif de distillation.

5. Procédé selon les revendications 1 à 4, dans lequel la distillation est réalisée en deux étapes, la pression lors de la première étape, mesurée au fond, étant au moins 1,5 fois la pression lors de la seconde étape, mesurée au fond, au moins 20 % en poids de la quantité introduite dans la première étape par unité de temps étant soutirée du fond de la première étape et introduite dans la seconde étape, et le produit de tête de la seconde étape étant recyclé dans la première étape.

6. Procédé selon les revendications 1 à 5, dans lequel la distillation est réalisée en présence de dioxyde de carbone.
